# EUROPEAN PATENT SPECIFICATION

(11) **EP 2 837 853 B1**
(45) Date of publication and mention of the grant of the patent: **26.10.2016**
(21) Application number: 13770271.8
(22) Date of filing: 28.03.2013
(51) Int. Cl.: F16J 13/12, F17C 1/16

(54) **PRESSURE VESSEL**
DRUCKBEHÄLTER
RECIPIENT A PRESSION

(30) Priority: 30.03.2012 JP 2012081782
(43) Date of publication of application: 18.02.2015
(73) Proprietor: Japan Agency for Marine-Earth Science and Technology, Yokosuka-shi, Kanagawa 237-0061 (JP); Toray Industries, Inc., Tokyo 103-8666 (JP)
(72) Inventor: ITO Hisao, Yokosuka-shi Kanagawa 237-0061 (JP); KATO Kazumasa, Yokosuka-shi Kanagawa 237-0061 (JP); OCHI Yutaka, Nagoya-shi Aichi 455-8502 (JP); HOSOKAWA Naofumi, Nagoya-shi Aichi 455-8502 (JP); HAGITA Hirohisa, Shimada-shi Shizuoka 427-8512 (JP); KAWAUCHI Shigekazu, Makinohara-shi Shizuoka 421-0506 (JP)
(74) Representative: Michalski Hüttermann & Partner Patentanwälte mbB
(86) International application number: PCT/JP2013/059360
(87) International publication number: WO 2013/147074

(56) References cited:
- DE-A1- 10 246 868
- JP-A- 2001 181 406
- JP-U- H 064 497
- JP-U- S5 143 408
- JP-U- S6 053 118
- JP-U- S6 417 996
- JP-U- S61 119 661

## Description

The present invention relates to a pressure vessel.

Thus far, as disclosed in JP 2009-121652, a pressure vessel comprising a CRFP (Carbon Fiber Reinforced Plastic) layer is proposed as a pressure vessel used for a fuel cell system, etc.

In the meanwhile, a pressure vessel is used for analyzing a rock sample, etc. collected by digging from an interior of an underground hole (e.g., for analyzing a cracking direction of a rock sample, etc.). In detail, a user stores the rock sample, etc. in the pressure vessel, maintains the interior of the pressure vessel at the same pressure as that of the interior of the underground hole, and then performs the analyzing using an X-ray Computed Tomography (CT) device. The pressure vessel disclosed in Patent Literature 1 has a liner installed at the inner side of a body portion made of a CRFP material. When the liner is made of a metal, the X-ray is damped in the liner, so that the analyzing using the X-ray cannot be properly performed. Thus, the pressure vessel disclosed in JP 2009-121652 is not suitable as a pressure vessel used for analyzing a rock sample, etc.

The body portion of the pressure vessel should be made of a material having the strength capable of enduring pressure and having a high X-ray transmittance. The metal material has a sufficient strength but has a low X-ray transmittance, so that the metal material is not suitable for the pressure vessel material.

A pressure vessel having a body portion formed of fiber reinforced plastic is described in DE10246868.

In general, the X-ray transmission rate increases as the atomic number of atoms constituting the material decreases and the density of the material decreases. An example of a material having the aforementioned property and high strength is a fiber reinforced plastic.

Thus, it is preferred that a main body of the pressure vessel used for analyzing a rock sample, etc. is made only of a fiber reinforced plastic. For example, it is preferred that a carbon-fiber pipe is used as a main body of the pressure vessel. When a carbon-fiber pipe is used as a main body of the pressure vessel, it is necessary that a member for blocking an opening portion, e.g., a metal-made flange, should be installed at both ends thereof. In an aircraft field, for example, a rivet is used for connection between the carbon-fiber pipe and a metal portion. In the case of the pressure vessel, a jointing area at the rivet is small, so that it is difficult for the rivet to endure use pressure resulting from high confining pressure which maintains the interior of the pressure vessel to be the same pressure as that of the underground hole.

Further, since the pressure vessel for the aforementioned usage should store the rock sample, etc. without damaging the shape (cylindrical shape) obtained by digging, it is necessary that at least one end thereof is largely opened. Further, when the outer diameter of the vessel is small, it is suitable to perform the analyzing, so that it is preferred that an opening having the same size as that of the inner diameter of the vessel is obtained. Thus, the pressure vessel for storing gas or liquid and having mirror portions at both ends thereof, disclosed in JP 2009-121652 is not suitable as a pressure vessel used for analyzing a rock sample, etc.

The present invention is conceived to solve the aforementioned problems, and the present invention is to provide a pressure vessel which can properly be used under a high confining pressure such as analysis a rock sample, etc. collected from the interior of an underground hole.

In order to solve the aforementioned problem, the pressure vessel according to the present invention includes: a tubular portion formed of a fiber reinforced plastic; and a cover portion provided at at least one end portion of the tubular portion, wherein an outer periphery of the end portion of the tubular portion has a reverse-tapered shape in which a diameter of the outer periphery is increased toward an end face, and the cover portion includes: a wedge-shaped member installed along the reverse-tapered shape of the outer periphery of the end portion of the tubular portion, and a cover portion main body portion for covering the wedge-shaped member from the outside and covering an opening of the tubular portion.

The pressure vessel according to an embodiment of the present invention has the tubular portion and the cover portion which are joined to each other using a reverse-taper shaped slope of the tubular portion, and has a structure which can endure pressure for separating the tubular portion and the cover portion even when the interior of the tubular portion maintains at high confining pressure. Further, the tubular portion formed of a fiber reinforced plastic to be joined to the cover portion formed of a metal material, etc. has only to have a reverse-tapered shape, and the pressure vessel can be configured while maintaining the strength of the tubular portion itself. Further, the tubular portion is formed of a fiber reinforced plastic, so that an object to be analyzed such as a rock sample, etc. stored in the pressure vessel can be analyzed without greatly reducing the X-ray. That is, in accordance with the pressure vessel according to an embodiment of the present invention, applications under a high confining pressure such as analysis a rock sample, etc. collected from the interior of an underground hole can be properly performed.

An angle of the reverse-tapered shape of the tubular portion with respect to an axial direction of the tubular portion may be an angle in a range of 3° to 5°. In accordance with this configuration, the end portion of the tubular portion, having a reverse-tapered shape, is designed to endure pressure for separating the tubular portion and the cover portion, so that the pressure vessel can endure a higher pressure from the inside.

Since a stress generated in the tubular portion by an inner pressure of the vessel includes a tensile stress in an axial direction of the tubular portion and a tensile stress in a circumferential direction of the tubular portion, reinforced fibers disposed at the tubular portion in order to improve the strength of the vessel may be oriented in two directions. However, when it is difficult to realize this configuration due to the limiting in a forming method, etc., the reinforced fibers may be oriented such that a slope from each of directions is equal to or smaller than 5°, due to a strength characteristic of when a tensile force in a diagonal direction is applied to a one-direction fiber reinforced plastic member which will be described below. Hereinafter, a case where the reinforced fibers have slopes as described above is also represented as extending in an axial direction or a circumferential direction.

A plurality of fibers substantially extending in an axial direction of the tubular portion is installed in the interior of the tubular portion, and the plurality of fibers are installed to have angles which become larger with respect to the axial direction of the tubular portion as they are installed to be closer to an outside in a radial direction in the end portion of the tubular portion. Otherwise, a plurality of fibers substantially extending in an axial direction of the tubular portion are installed in the interior of the tubular portion, and the plurality of fibers are installed such that an angle with respect to a radial direction of the tubular portion in the end portion of the tubular portion is equal to the reverse-tapered shape. In accordance with this configuration, the strength of the end portion of the tubular portion can be secured, so as to surely implement the present invention.

The cover portion main body portion has a hole installed therein of which one opening is connected to an opening of the tubular portion, and the cover portion is detachably installed at the other opening of the hole of the cover portion main body portion, and may further include a cap for blocking the other opening. In accordance with this configuration, an object to be analyzed, etc. can be easily stored in the interior of the pressure vessel.

The cover portion may have a configuration which can apply pressure to the interior of the tubular portion covered by the cover portion. In accordance with this configuration, the interior of the pressure vessel can be easily maintained at a high pressure.

The tubular portion may be made of a carbon fiber reinforced plastic. The cover portion may be made of a metal material.

In accordance with an embodiment of the present invention, applications under a high confining pressure such as analysis a rock sample, etc. collected from the interior of an underground hole can be properly performed.
Fig. 1 is (a) a front view, (b) a side view and (c) a sectional view illustrating a pressure vessel according to the present invention;
Fig. 2 is (a) a front view, (b) a side view (c) a sectional view and (d) a perspective view illustrating a tubular portion and (e) a front view illustrating an end portion of the tubular portion;
Fig. 3 illustrates an arrangement of a fiber at the end of the tubular portion;
Fig. 4 is (a) a front view, (b) a side view and (c) a sectional view illustrating a wedge-shaped member;
Fig. 5 is (a) a front view, (b) a side view and (c) a sectional view illustrating an outer ring;
Fig. 6 is (a) a front view, (b) a side view, (c) a sectional view and (d) a perspective view illustrating an inner ring;
Fig. 7 is (a) a front view, (b) a side view and (c) a sectional view illustrating a fixing screw of the outer ring;
Fig. 8 is (a) a front view, (b) a side view, (c) a sectional view and (d) a perspective view illustrating a sample cap;
Fig. 9 is (a) a front view, (b) a side view, (c) a sectional view and (d) a perspective view illustrating a sample cap screw;
Fig. 10 is (a) a front view, (b) a side view and (c) a sectional view illustrating a sample cap fixing pin; and
Fig. 11 is a graph depicting a strength characteristic of when a tensile force is applied to a one-direction fiber reinforced plastic member in a diagonal direction.

Hereinafter, an embodiment of a pressure vessel according to the present invention together with the accompanying drawings will be described in detail. Further, in the description of the accompanying drawings, the same elements will be designated by the same reference numerals as far as possible and duplicate descriptions thereof will be omitted. Further, the dimension ratios of the accompanying drawings may not always coincide with the description.

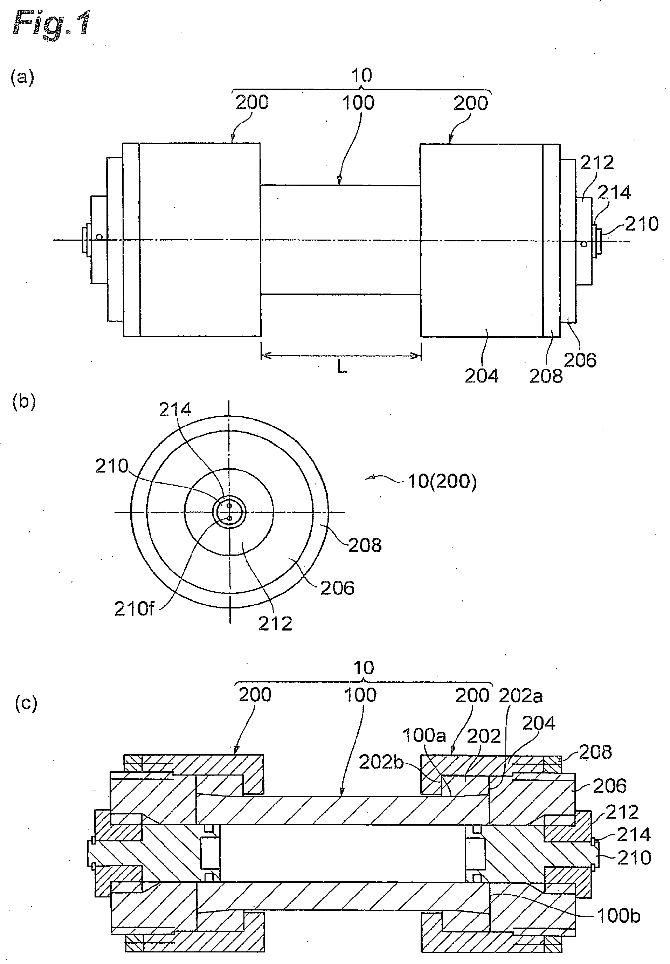

Fig. 1 is (a) a front view, (b) a side view and (c) a sectional view (taken along a central line of Fig. 1(a)) illustrating a pressure vessel 10 according to the present embodiment. As illustrated in Figs. 1, a pressure vessel 10 includes a tubular portion 100 (confining pressure pipe), and cover portions (flanges) 200 connected to both ends of the tubular portion 100 to block openings at the both ends of the tubular portion 100.

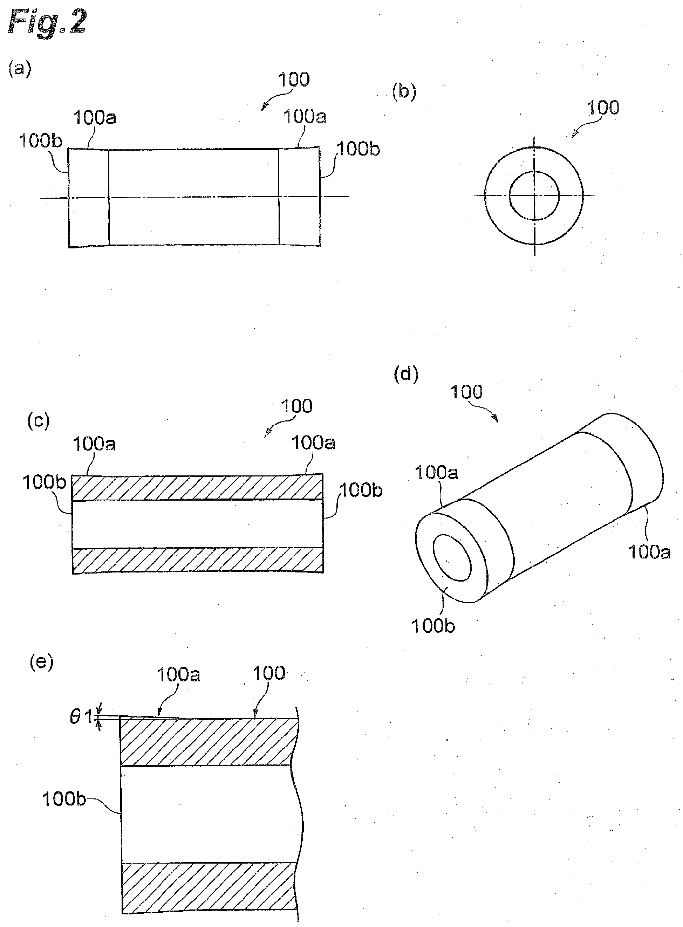

Fig. 2 is (a) a front view, (b) a side view (c) a sectional view and (d) a perspective view illustrating the tubular portion 100, and (e) a front view illustrating an end portion of the tubular portion 100. As illustrated in Fig. 1 and Fig. 2, the tubular member 100 is a member having, for example, a cylindrical shape. The tubular portion 100 constitutes the interior of the pressure vessel 10, and receives a thing stored (enclosed) in the pressure vessel 10 (e.g., a rock sample, etc. collected by digging from the interior of the underground hole). The inner diameter and the length of the tubular portion 100 may be arbitrary size according to a thing to be stored in the pressure vessel 100 and a necessary strength. For example, when the tubular portion 100 is used for storing a rock sample, etc., the inner diameter of the central portion in an axial direction thereof may be 72 mm, and the axial length L between the cover portions 200 at the both ends thereof may be 200 mm.

The tubular portion 100 is a member made of a fiber reinforced plastic. For example, a CRFP is employed as the fiber reinforced plastic. Otherwise, for example, GRFP (Glass Fiber Reinforced Plastic), AFRP (Aramid Fiber Reinforced Plastic), or the like may be also employed. In order to improve the strength of the vessel, fibers in the tubular portion 100 may be oriented in two directions including an axial direction thereof and a circumferential direction thereof.

As illustrated in Fig. 1 and Fig. 2, the outer peripheral surface of an end portion 100a of the tubular portion 100 has a reverse-tapered shape in which the diameter thereof is increased toward an end face 100b. The reversed-tapered shape aims to fix the cover portions 200 to both ends of the tubular portion 100 as described below. The end portion 100a of the tubular portion 100, having the reverse-tapered shape, is a portion extending in an axial direction from the end face 100b by a predetermined length, and is, for example, a portion extending from both end face 100b by 60 mm, respectively. It is preferred that a taper angle θ1 of the reverse-tapered shape of the end portion 100a (angle with respect to an axial direction of the tubular portion 100) may be set as being in a range of 3° to 5° in consideration of a fixing strength between the tubular portion 100 and the cover portion 200, and for example, it is set as an angle of 3°. However, when the strength meeting a purpose can be secured, the angle θ1 needs not always be set as the above angle.

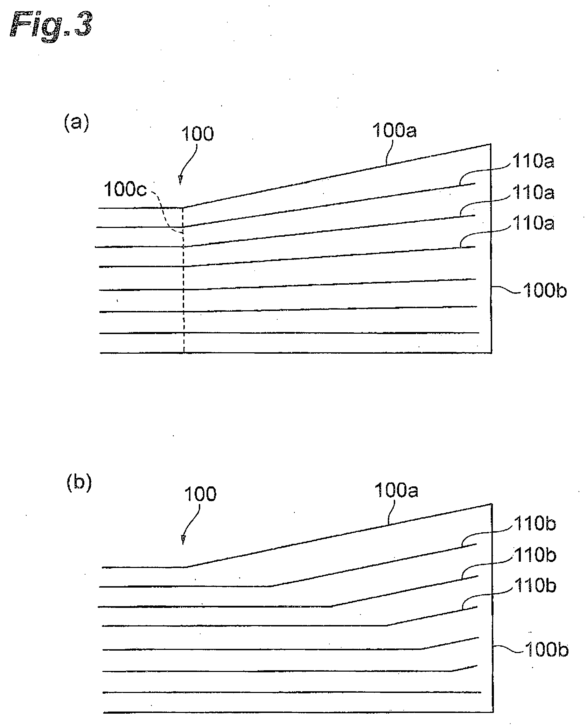

Fig. 3 illustrates arrangements of fibers in the end portion 100a of the tubular portion 100, the fibers extending in an axial direction of the tubular portion 100. Examples of the arrangement of the fibers in the end portion 100a of the tubular portion 100 may be an arrangement illustrated in Fig. 3(a), or an arrangement illustrated in Fig. 3(b).

In the arrangement illustrated in Fig. 3(a), fibers 110a substantially extending in an axial direction of the tubular portion 100 are installed to have angle which become larger with respect to the axial direction of the tubular portion 100 as they are installed to be closer to an outside in a radial direction in the end portion 100a of the tubular portion 100. In this embodiment, the fibers 110a installed in the axial direction are bent in the same direction as that of the reverse-tapered shape, at a boundary line 100c with a portion having the reverse tapered shape in the axial direction. Further, each of the fibers 110a has a large angle which is to be close to a taper angle of the end portion 100a as it is installed to be closer to the outside in a radial direction.

In the arrangement illustrated in Fig. 3(b), fibers 110b substantially extending in an axial direction of the tubular portion 100 are installed such that an angle with respect to an axial direction of the tubular portion 100 in the end portion 100a of the tubular portion 100 is the same as that of the reverse tapered shape. In this embodiment, the fibers 110b installed in the axial direction are bent at the same angle (taper angle) as that of the reverse-tapered shape at a portion closer to the end face 100b as they are installed to be closer to an inner side.

The arrangement method of the fibers 110a and 110b illustrated in Fig. 3(a) and Fig. 3(b) can secure the strength of the tubular portion 100, which is sufficient for the pressure vessel 10.

The cover portion 200 installed at the both ends of the tubular portion 100 includes a wedge-shaped member 202, an outer ring 204, an inner ring 206, an outer ring fixing screw 208, a sample cap 210, a sample cap screw 212, and a sample cap fixing pin 214. Each of members constituting the cover portions 200 is made of a metal material. Information on which metal forms the members will be described in detail below.

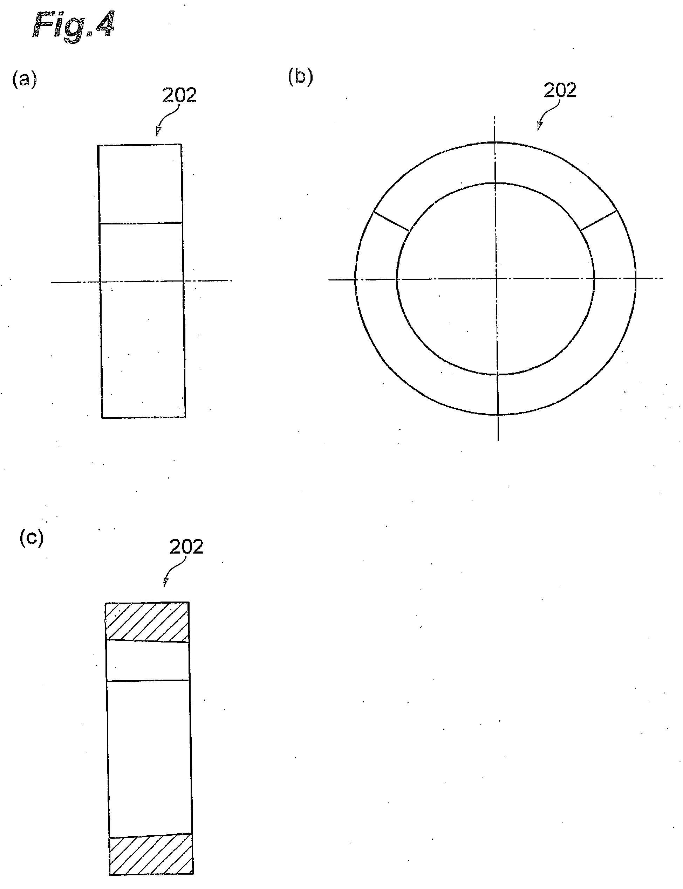

The wedge-shaped member 202 is a member installed to be in close contact with the end portion 100a of the tubular portion 100 along the reverse-tapered shape of an outer peripheral surface thereof. The wedge-shaped member 202 is a circular arc member, and is a plurality of members (for example, three members) obtained by dividing a cylindrical (circular annular) member, as illustrated in the front view of Fig. 4(a), the side view of Fig. 4(b), and the sectional view of Fig. 4(c). The axial length of the wedge-shaped member 202 is the same as the axial length of the reverse-tapered end portion 100a of the tubular portion 100.

Further, the inner periphery of the wedge-shaped member 202 has a tapered shape to be installed in close contact with the reverse-tapered shape. That is, the diameter of one end 202a of the inner periphery of the wedge-shaped member 202 is the same as that of the end face 100b of the tubular portion 100, and the diameter of the other end 202b is the same as that of the central portion (portion formed not to have a tapered shape) of the tubular portion 100. Further, it is preferred that the outer periphery of the wedge-shaped member 202 maintains the same size across the axial direction.

For example, the axial length of the wedge-shaped member 202 may be 60 mm, and the outer diameter thereof may be 200 mm. It is preferred that the wedge-shaped member 202 is made of a material which is softer than the outer ring 204 and the inner ring 206 covering the wedge-shaped member 202, e.g. a copper alloy, since the wedge-shaped member 202 function as a wedge literally.

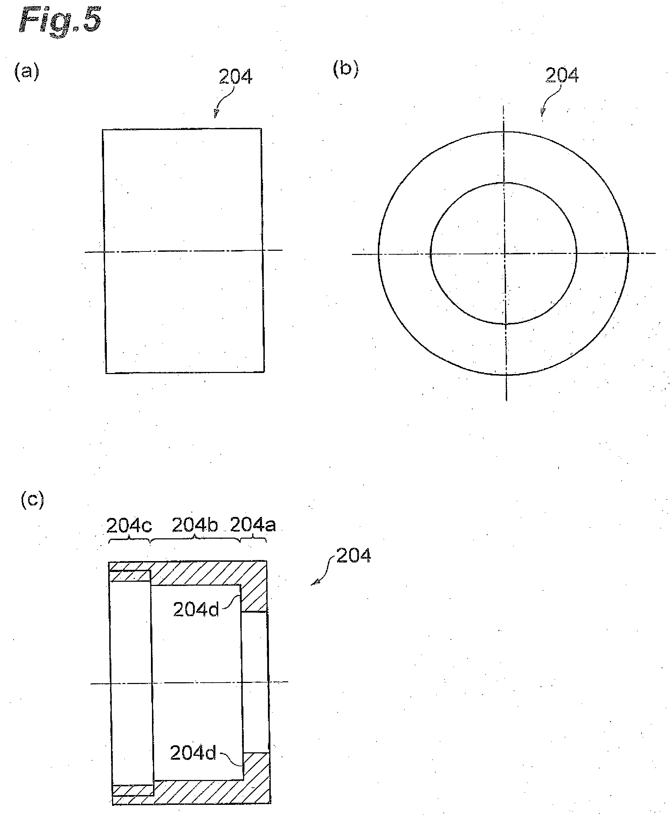

The outer ring 204 is the cover portion outer peripheral portion (one component of a cover portion main body portion) covering the wedge-shaped member 202 from the outside. Fig. 5 is (a) a front view, (b) a side view and (c) a sectional view illustrating the outer ring 204. The outer ring 204 is a cylindrical member, and the outer diameter thereof is designed to be larger than the outer diameter of the wedge-shaped member 202 to cover the wedge-shaped member 202. The outer ring 204 may be divided into an inner portion 204a, an intermediate portion 204b, and an outer portion 204c according to the size of the inner diameter in the axial direction. The inner portion 204a is installed at a central portion in the axial direction of the tubular portion 100. The inner portion 204a, the intermediate portion 204b, and the outer portion 204c are sequentially located from the central portion in the axial direction of the tubular portion 100 toward the outside.

The inner diameter of the inner portion 204a of the outer ring 204 is designed to be equal to or larger than the outer diameter of the end face 100b of the tubular portion 100 (the tubular portion 100 has a reverse-tapered shape, so that the outer diameter thereof is larger than the central portion in the axial direction of the tubular portion 100) to pass through the tubular portion 100.

A surface 204d at the inner side of the inner portion 204a is in contact with the end face 202b (surface of which the inner periphery has the same diameter as that of the central portion of the tubular portion 100) at the central portion of the tubular portion 100 in the axial direction of the wedge-shaped member 202. Thus, the inner diameter of the inner portion 204a is smaller than the outer diameter of the wedge-shaped member 202. Further, when pressure is applied to the interior of the pressure vessel 10, a force is applied to the surface 204d at the inner side of the inner portion 204a toward the end face 202b of the wedge-shaped member 202 by the pressure, so that the thickness and the size of the diameter of the inner portion 204a is designed to endure the force.

The intermediate portion 204b of the outer ring 204 is a portion located at the outer side of the radial direction of the wedge-shaped member 202. Thus, the inner periphery surface of the intermediate portion 204b is installed in close contact with the outer peripheral surface of the wedge-shaped member 202. Accordingly, the inner diameter of the intermediate portion 204b has the same as the outer diameter of the wedge-shaped member 202. Further, the axial length of the intermediate portion 204b becomes longer than the axial length of the wedge-shaped member 202.

The outer portion 204c of the outer ring 204 is a portion which is fixed to the inner ring 206. The diameter of the inner periphery of the outer portion 204c is designed to be larger than the diameter of the inner periphery of the intermediate portion 204b. The outer ring 204 and the inner ring 206 are fixed to each other by screw clamp. The screw groove is installed at the inner periphery of the outer portion 204c. Further, in the pressure vessel 10, a screw-clamped portion and a contact-fixed portion may be bonded using an adhesive such that the contact strength becomes stronger.

For example, the outer diameter of the outer ring 204 is 250 mm, and the axial length of the outer portion 204c is 40 mm. The outer ring 204 is formed by, for example, a mechanical structure steel material.

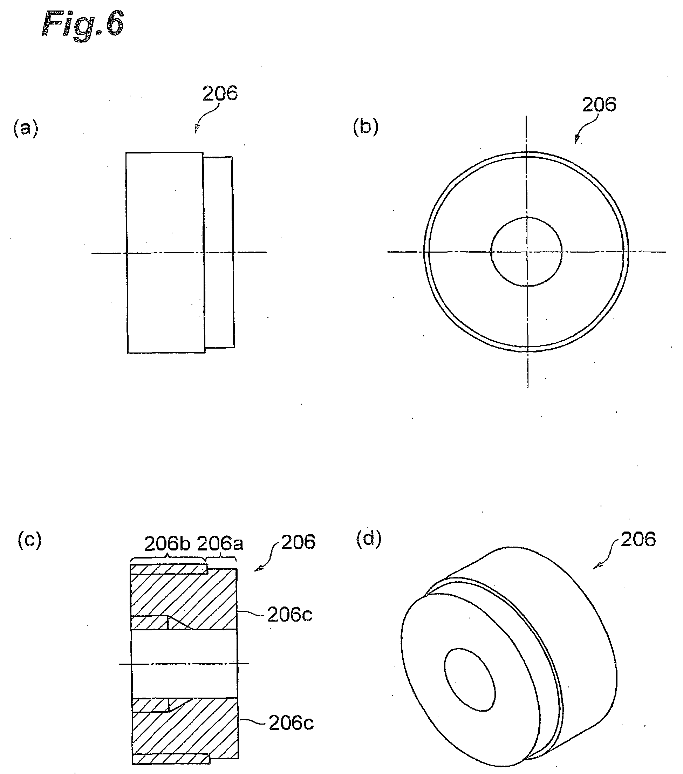

The inner ring 206 is one component of the cover portion main body portion which is installed to cover the openings of the tubular portion 100 and the wedge-shaped member 202, from the opening side of the tubular portion 100, and comes into contact with the outer ring 204. Fig. 6 is (a) a front view, (b) a side view, (c) a sectional view and (d) a perspective view illustrating the inner ring 206. The inner ring 206 is a cylindrical member, and the size of the inner diameter thereof is the same as that of the inner diameter of the tubular portion 100. The inside cylinder of the inner ring 206 is connected to the inside cylinder of the tubular portion 100, and becomes a take-in/out opening for things stored in the pressure vessel 10. That is, a hole of which one opening is connected to the opening of the tubular portion 100 is installed in the inner ring 206. The inner ring 206 may be divided into an inner portion 206a and an outer portion 206b according to the size of the outer diameter in the axial direction. The inner portion 206a is installed at the tubular portion 100 side.

The inner ring 206 and the outer ring 204 are fixed (connected) to each other by screw clamp, so that an end face 206c of the inner portion 206a of the inner ring 206 collides with the end face 100b of the tubular portion 100. Accordingly, as illustrated in Fig. 1(c), the wedge-shaped member 202 becomes a close contact state by the outer ring 204 and the end portion 100a of the tubular portion 100, the end portion 100a having a reverse-tapered shape.

A screw groove for fixing the outer ring 204 and the inner ring 206 is installed at the outer portion 206b of the inner ring 206. The axial length of the outer portion 206b is longer than the length of the outer portion 204c of the outer ring 204 in order to fit the outer ring fixing screw 208 for fixing the outer ring 204 from a further outer side. A screw groove for screw clamp a sample cap screw 212 is installed at an opening side of the inner periphery of the outer portion 206b of the inner ring 206. The inner ring 206 is formed by, for example, a mechanical structure steel material.

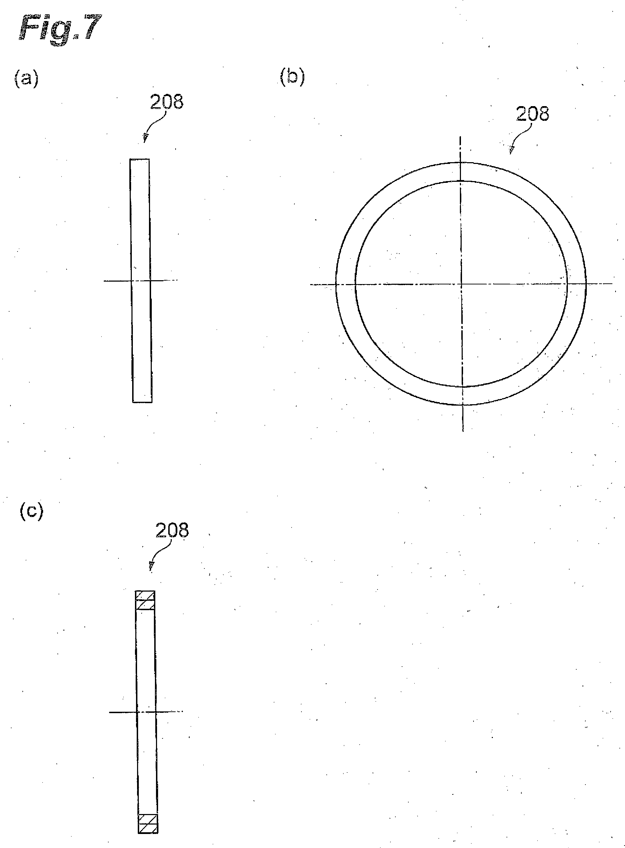

The outer ring fixing screw 208 is a screw for fixing the outer ring. Fig. 7 is (a) a front view, (b) a side view and (c) a sectional view illustrating the outer ring fixing screw 208. The outer ring fixing screw 208 is a cylindrical (circular-annular) member, and the outer diameter thereof has the same as that of the outer ring 204. A screw groove for screw clamp (the outer periphery of the outer portion 206b of) the inner ring 206 and the outer ring 208 is installed at the inner periphery of the outer ring fixing screw 208. The outer ring fixing screw 208 is screw clamped to the inner ring 206, at a location which is in close contact with the outer ring 204, in order to fix the outer ring 204 not to move in the axial direction.

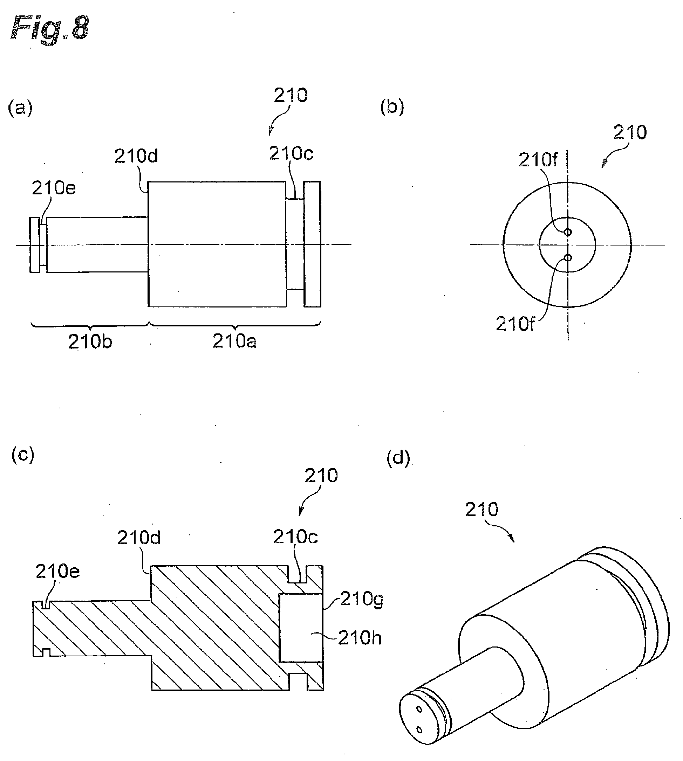

The sample cap 210 is a cap detachably installed at the opening at the outside of the hole of the inner ring 206 (opening at the outer portion 206b side of the inner ring 206) to block the opening. Fig. 8 is (a) a front view, (b) a side view (view when viewed from the left side of the Fig. 8(a)), (c) a sectional view and (d) a perspective view illustrating the sample cap 210. The sample cap 210 is a columnar member. The sample cap 210 may be divided into an inner portion 210a and an outer portion 210b according to the size of the outer diameter in the axial direction. The inner portion 210a is installed at a central side of the tubular portion 100.

The inner portion 210a of the sample cap 210 is a portion for blocking the opening of the tubular portion 100. A groove 210c capable of receiving a sealing member such as an O-ring is installed in the inner portion 210a to surely block the tubular portion 100. Further, the sealing by the inner portion 210a is directly performed at the inner periphery of the tubular portion 100.

The outer portion 210b of the sample cap 210 is a portion to which the sample cap screw 212 for fixing the sample cap 210 to the inner ring is connected. The outer diameter of the outer portion 210b is designed to be smaller than the inner portion 210a, and the sample cap screw 212 is fixed by colliding with an end face 210d at the outer portion 210b side of the inner portion 210a. Further, a groove 210e capable of receiving the sample cap fixing pin 214 for preventing separation of the sample cap screw 212 is installed at an end portion at the end face side of the outer portion 210b.

Small holes 210f having openings at both ends are installed at the sample cap 210. When the sample cap 210 is fixed to the inner ring 206, one opening of these holes 210f is located at the outside of the pressure vessel 10, and the other opening is located at the inside (of the tubular portion 100) of the pressure vessel 10. The opening at the outside serves as a connection portion connected with a pressure system. The pressure system is connected to the opening at the outside, and fluid such as water flows to the interior (of the tubular portion 100) of the pressure vessel 10 through the holes 210f from the opening, so that pressure can be applied to the interior of the pressure vessel 10. That is, the cover portion 200 is configured to apply pressure to the interior (of the tubular portion 100) of the pressure vessel 10.

Further, a depressed portion 210h is installed at an end face 210g of the inner portion 210a of the sample cap 210. The depressed portion 210h is formed such that a shape of an cross-section surface perpendicular to the axial direction becomes circular (that is, a shape which extends toward the inside to be columnar). For example, a columnar rod formed of plastic is inserted into the depressed portion 210h, so that location the things stored in the pressure vessel 10 can be determined or fixed.

For example, the outer diameter of the inner portion 210a of the sample cap 210 may be 72 mm in accordance with the inner diameter of the tubular portion 100. The sample cap 210 is formed of, for example, a stainless steel.

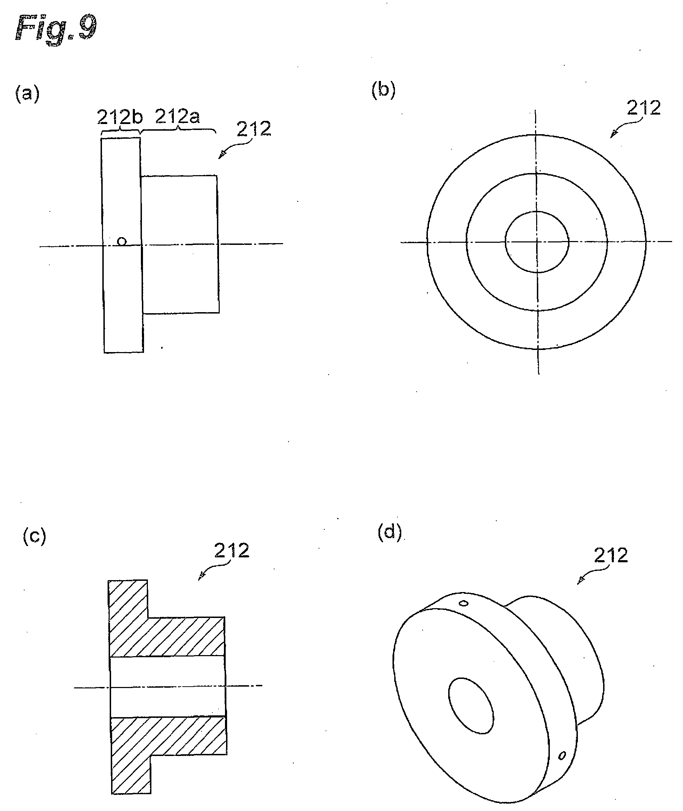

The sample cap screw 212 is a member for detachably fixing the sample cap 210 to the inner ring 206. Fig. 9 is (a) a front view, (b) a side view, (c) a sectional view and (d) a perspective view illustrating the sample cap screw 212. The sample cap screw 212 is a cylindrical member, and the outer portion 210b of the sample cap 210 is inserted into a hole of the sample cap screw 212. Further, the axial direction length of the sample cap screw 212 is shorter than the length between the end face 210d at the outer portion 210b side of the inner portion 210a of the sample cap 210 and a groove 210e of the outer portion 210b such that the sample cap fixing pin 214 is mounted to the sample cap 210. The sample cap screw 212 may be divided into an inner portion 212a and an outer portion 212b according to the size of the outer diameter in the axial direction. The inner portion 212a is installed at the tubular portion 100 side.

The inner portion 212a of the sample cap screw 212 has a screw groove installed therein in order to be fixed to an opening side of the inner periphery of the outer portion 206b of the inner ring 206 by screw clamp. The outer diameter of the outer portion 210b of the sample cap screw 212 is designed to be larger than the outer diameter of the inner portion 212a. The sample cap screw 212 is formed of, for example, a nickel alloy.

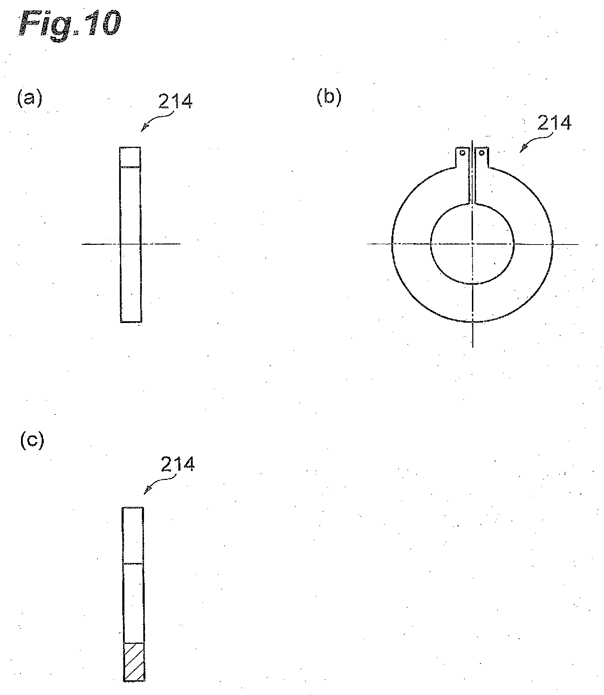

The sample cap fixing pin 214 is a member for preventing separation of the sample cap screw 212 from the sample cap 210. Fig. 10 is (a) a front view, (b) a side view and (c) a sectional view illustrating the sample cap fixing pin 214. The sample cap fixing pin 214 is a cylindrical (circular annular) member. The sample cap fixing pin 214 is received in the groove 210e of the sample cap 210 while the sample cap screw 212 is inserted into the outer portion 210b of the sample cap 210, so as to prevent separation of the sample cap screw 212 from the sample cap 210. Meanwhile, the sample cap 210, the sample cap screw 212 and the sample cap fixing pin 214 are components of the cover portion main body portion for covering (blocking) the opening of the tubular portion 100. Hereinabove, the pressure vessel 10 according to the present embodiment has been described.

As described above, in the pressure vessel 10 according to the present embodiment, the tubular portion 100 and the cover portion 200 are joined to each other using a reverse-tapered shaped slope of the tubular portion 100. In detail, the wedge-shaped member 202 is installed at a portion enclosed by the end portion 100a of the tubular portion 100, having a reverse-tapered shape, and the outer ring 204 and the inner ring 206 of the cover portion 200. Further the tubular portion 100 and the cover portion 200 is joined by the wedge-shaped member 202. When a high confining pressure is applied to the interior of the tubular portion 100, a pressure which separates the tubular portion 100 and the cover portion 200 from each other is generated. However, since the wedge-shaped member 202 is installed, the pressure vessel 10 can endure the pressure.

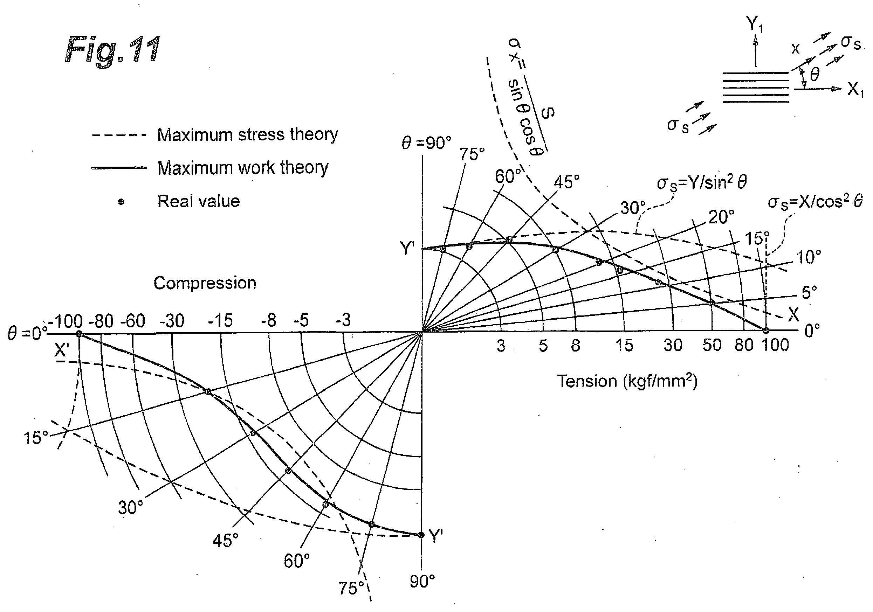

Here, Fig. 11 is a graph depicting a strength characteristic of when a tensile force is applied to a one-direction fiber reinforced plastic member in a diagonal direction. This graph is a graph disclosed on an 89th page of Fujii Taichi, Zako Masaru, Fracture and Mechanics of Composite Material, Jikkyo Shuppan Co., Ltd., 1978. In this graph, when viewing a graph of the maximum work theory which accords well with a real value, the tensile strength at an angle of 5° is a half of the tensile strength at an angle of 0°, and the tensile strength at an angle of 3° is 70% of the tensile strength at an angle of 0°. Considering this fact, a tapered angle of the reverse-tapered shape of the tubular portion 100 may be 3° to 5° as described above, and a slope from the axial direction and the circumferential direction of the reinforced fiber arranged in a tubular portion may be 5° or lower.

Further, the tubular portion 100 formed of a fiber reinforced plastic to be joined to the cover portion 200 formed of a metal material, etc. has only to have the end portion 100a having a reverse-tapered shape. Thus, a special shape obtained by weakening the strength in order to join the tubular portion 100 to the cover portion 200 is unnecessary, and the pressure vessel 10 may be configured while maintaining the strength of the tubular portion 100 itself. Further, the tubular portion 100 is formed of a fiber reinforced plastic, so that an object to be analyzed such as a rock sample, etc. stored in the pressure vessel 10 can be analyzed without greatly reducing the X-ray. Further, when a metal pressure vessel is used, the weight thereof is heavy, so that it is difficult to simply mount the metal pressure vessel to an X-ray CT device. However, the weight of the pressure vessel is reduced by using the tubular portion 100 formed of fiber reinforced plastics. That is, in accordance with the pressure vessel 10 according to the present embodiment, applications under a high confining pressure such as analysis a rock sample, etc. collected from the interior of an underground hole can be properly performed.

In detail, the pressure vessel 10 according to the aforementioned embodiment can maximally endure a pressure of 200 MPa. Further, the pressure vessel 10 can be used maximally under a temperature of 100 °C.

Further, the cover portions 200 at both ends have a metal flange structure as in the present embodiment, so that a storage object such as a rock sample, etc. can be easily taken in or out. Further, when the detachable sample cap 210 is installed in the cover portion 200 as in the present embodiment, the storage object can be more easily taken in or out of the pressure vessel 10. Further, when pressure can be applied to the interior of the pressure vessel 10 as in the present embodiment, the interior of the pressure vessel can easily maintain high confining pressure.

Further, although the pressure vessel 10 according to the present embodiment has the cover portions 200 installed at the both ends of the tubular portion 100 and having a metal flange structure, the cover portion 200 having the metal flange structure may be installed at at least one end portion of the tubular portion 100. In this case, in the tubular portion 100, only the end portion 100a at a side where the cover portion 200 is installed has a reverse-tapered shape. Further, in the present embodiment, the tubular portion 100 has a configuration in which the both end portions 100a are opened. However, the tubular portion 100 may have a configuration in which only one end portion is opened. In this case, the opened end portion 100a has a reverse-tapered shape, and the cover portion 200 is installed at the end portion 100a.

In accordance with an embodiment of the present invention, applications under a high confining pressure such as analysis a rock sample, etc. collected from the interior of an underground hole can be properly performed.
- 10: Pressure vessel
- 100: Tubular portion
- 110a, 110b: Fiber
- 200: Cover portion
- 202: Wedge-shaped member
- 204: Outer ring
- 206: Inner ring
- 208: Outer ring fixing screw
- 210: Sample cap
- 212: Sample cap screw
- 214: Sample cap fixing pin

## Claims

1. A pressure vessel comprising:
a tubular portion (100) formed of a fiber reinforced plastic; and
a cover portion (200) provided at at least one end portion (100a) of the tubular portion (100),
**characterized in that** an outer periphery of the end portion (100a) of the tubular portion (100) has a reverse-tapered shape in which a diameter of an outer periphery thereof is increased toward an end face (100b), and
the cover portion (200) comprises:
a wedge-shaped member (202) installed along the reverse-tapered shape of the outer periphery of the end portion (100a) of the tubular portion (100), and
a cover portion main body portion for covering the wedge-shaped member (202) from the outside and covering an opening of the tubular portion (100).

2. The pressure vessel according to claim 1, wherein an angle of the reverse-tapered shape of the tubular portion (100) with respect to an axial direction of the tubular portion (100) is in a range of 3° to 5°.

3. The pressure vessel according to claim 1 or 2, wherein a plurality of fibers substantially extending in an axial direction of the tubular portion (100) are installed in an interior of the tubular portion (100), and
the plurality of fibers are installed to have angles which become larger with respect to the axial direction of the tubular portion (100) as they are installed to be closer to an outside in a radial direction in the end portion (100a) of the tubular portion (100).

4. The pressure vessel according to claim 1 or 2, wherein a plurality of fibers substantially extending in an axial direction of the tubular portion (100) is installed in an interior of the tubular portion (100), and
the plurality of fibers are installed such that an angle with respect to an axial direction of the tubular portion (100) in the end portion (100a) of the tubular portion (100) is equal to the reverse-tapered shape.

5. The pressure vessel according to one of claims 1 to 4, wherein the cover portion main body portion has a hole installed therein of which one opening is connected to an opening of the tubular portion (100), and
the cover portion (200) is detachably installed at the other opening of the hole of the cover portion main body portion, and further comprises a cap (210) for blocking the other opening.

6. The pressure vessel according to one of claims 1 to 5, wherein the cover portion (200) has a hole, and is configured to apply fluid or pressure to the interior of the tubular portion (100) covered by the cover portion (200).

7. The pressure vessel according to one of claims 1 to 6, wherein the tubular portion (100) is made of a carbon fiber reinforced plastic.

8. The pressure vessel according to one of claims 1 to 7, wherein the cover portion (200) is made of a metal material.

## Patentansprüche

1. Druckbehälter, mit:
einem aus einem faserverstärkten Kunststoff ausgebildeten rohrförmigen Abschnitt (100); und
einem an mindestens einem Endabschnitt (100a) des rohrförmigen Abschnitts (100) bereitgestellten Abdeckungsabschnitt (200),
**dadurch gekennzeichnet, dass**
ein Außenumfang des Endabschnitts (100a) des rohrförmigen Abschnitts (100) eine umgekehrt konische Form hat, gemäß der ein Durchmesser eines Außenumfangs davon zu einer Endfläche (100b) hin zunimmt; und
der Abdeckungsabschnitt (200) aufweist:
ein entlang der umgekehrt konischen Form des Außenumfangs des Endabschnitts (100a) des rohrförmigen Abschnitts (100) installiertes keilförmiges Element (202); und
einen Abdeckungsabschnitthauptkörperabschnitt zum Abdecken des keilförmigen Elements (202) von außen und zum Abdecken einer Öffnung des rohrförmigen Abschnitts (100).

2. Druckbehälter nach Anspruch 1, wobei ein Winkel der umgekehrt konischen Form des rohrförmigen Abschnitts (100) bezüglich einer axialen Richtung des rohrförmigen Abschnitts (100) im Bereich von 3° bis 5° liegt.

3. Druckbehälter nach Anspruch 1 oder 2, wobei
mehrere Fasern, die sich im Wesentlichen in einer axialen Richtung des rohrförmigen Abschnitts (100) erstrecken, in einem Innenraum des rohrförmigen Abschnitts (100) installiert sind, und
die mehreren Fasern derart installiert sind, dass ihre Winkel bezüglich der axialen Richtung des rohrförmigen Abschnitts (100) umso größer werden, je näher sie in einer radialen Richtung des Endabschnitts (100a) des rohrförmigen Abschnitts (100) an einer Außenseite installiert sind.

4. Druckbehälter nach Anspruch 1 oder 2, wobei
mehrere Fasern, die sich im Wesentlichen in einer axialen Richtung des rohrförmigen Abschnitts (100) erstrecken, in einem Innenraum des rohrförmigen Abschnitts (100) installiert sind, und
die mehreren Fasern derart installiert sind, dass ein Winkel bezüglich einer axialen Richtung des rohrförmigen Abschnitts (100) im Endabschnitt (100a) des rohrförmigen Abschnitts (100) der umgekehrt konischen Form angepasst ist.

5. Druckbehälter nach einem der Ansprüche 1 bis 4, wobei
im Abdeckungsabschnitthauptkörperabschnitt ein Loch ausgebildet ist, wobei eine Öffnung des Lochs mit einer Öffnung des rohrförmigen Abschnitts (100) kommuniziert, und
der Abdeckungsabschnitt (200) an der anderen Öffnung des Lochs des Abdeckungsabschnitthauptkörperabschnitts lösbar installiert ist und ferner eine Kappe (210) zum Blockieren der anderen Öffnung aufweist.

6. Druckbehälter nach einem der Ansprüche 1 bis 5, wobei der Abdeckungsabschnitt (200) ein Loch aufweist und dafür konfiguriert ist, dem Innenraum des durch den Abdeckungsabschnitt (200) abgedeckten rohrförmigen Abschnitts (100) ein Fluid oder Druck zuzuführen.

7. Druckbehälter nach einem der Ansprüche 1 bis 6, wobei der rohrförmige Abschnitt (100) aus einem kohlenstofffaserverstärkten Kunststoff hergestellt ist.

8. Druckbehälter nach einem der Ansprüche 1 bis 7, wobei der Abdeckungsabschnitt (200) aus einem Metallmaterial hergestellt ist.

## Revendications

1. Récipient sous pression comprenant :
une partie tubulaire (100) formée dans une matière plastique renforcée de fibres ; et
une partie de couvercle (200) mise en place à au moins une partie d'extrémité (100a) de la partie tubulaire (100),
**caractérisé en ce que** la périphérie extérieure de la partie d'extrémité (100a) de la partie tubulaire (100) a une forme de cône inverse dans laquelle un diamètre d'une périphérie extérieure de celui-ci est augmenté en direction d'une face d'extrémité (100b), et
la partie de couvercle (200) comprend :
un organe en forme de coin (202) installé le long de la forme en cône inverse de la périphérie externe de la partie d'extrémité (100a) de la partie tubulaire (100), et
une partie de corps de partie de couvercle pour recouvrir l'organe en forme de coin (202) à partir de l'extérieur et en recouvrant une ouverture de la partie tubulaire (100).

2. Récipient sous pression selon la revendication 1, dans lequel un angle de la forme en cône inverse de la partie tubulaire (100) par rapport à une direction axiale de la partie tubulaire (100) est dans une plage de 3 ° à 5 °.

3. Récipient sous pression selon la revendication 1, ou 2, dans lequel une pluralité de fibres s'étendant essentiellement dans une direction axiale de la partie tubulaire (100) est placée dans une région intérieure de la partie tubulaire (100), et
la pluralité des fibres est placée afin de présenter des angles qui deviennent plus grands par rapport à la direction axiale de la partie tubulaire (100) lorsqu'elles sont placées pour être plus proches vis à vis d'une région extérieure dans une direction radiale dans la partie d'extrémité (100) de la partie tubulaire (100).

4. Récipient sous pression selon la revendication 1, ou 2, dans lequel une pluralité de fibres s'étendant essentiellement dans une direction axiale de la partie tubulaire (100) est placée dans une région intérieure de la partie tubulaire (100), et
la pluralité des fibres est placée de telle manière qu'il y ait un angle par rapport à une direction axiale de la partie tubulaire (100) dans la partie d'extrémité (100) de la partie tubulaire (100) égal à la forme en cône inverse.

5. Récipient sous pression selon l'une des revendications 1 à 4, dans lequel la partie de corps de la partie de couvercle a un trou y étant installé, dont une ouverture est en communication avec une ouverture de la partie tubulaire (100), et
la partie de couvercle (200) est installée détachable à l'autre ouverture du trou de la partie de corps de la partie de couvercle, et comprend en outre un capuchon (210) pour le blocage de l'autre ouverture.

6. Récipient sous pression selon l'une des revendications 1 à 5, dans lequel la partie de couvercle (200) a un trou, et est configurée pour appliquer un fluide ou une pression à l'intérieur de la partie tubulaire (100) recouverte par la partie de couvercle (200).

7. Récipient sous pression selon l'une des revendications 1 à 6, dans lequel la partie tubulaire (100) est fabriquée dans une matière plastique renforcée de fibres de carbone.

8. Récipient sous pression selon l'une des revendications 1 à 7, dans lequel la partie de couvercle (200) est fabriquée dans un matériau métallique.
